# EUROPEAN PATENT APPLICATION

(11) **EP 1 832 854 A1**
(43) Date of publication of application: **12.09.2007**
(21) Application number: 06122455.6
(22) Date of filing: 17.10.2006
(51) Int. Cl.: G01F 1/28, A61M 16/04, A61M 25/00

(54) **Cannula gas flow meter**

(30) Priority: 14.09.2006 US 522005; 10.03.2006 US 780980 P
(71) Applicant: INGEN TECHNOLOGIES, INC., 92320 CA Calimesa (US)
(72) Inventor: McDermott, Francis, Yucaipa, CA 92399 (US); Campbell, Richard G., Corona, CA 92882 (US)
(74) Representative: Viering, Jentschura & Partner

(57) **Abstract**

A meter for visual determination of proper oxygen or gas flow to a facemask or cannula from a pressurized gas supply. The device features a body with a central cavity extending axially therein adapted for translation of a piston biased toward an incoming gas stream from the pressurized supply. A cup-shaped endwall of the piston increases the area on which the incoming air stream acts. The incoming air stream is focused to impact a center portion of the endwall at a higher rate of speed using a narrowing incoming conduit resulting in increased force and translation of the piston even under the low pressures in which cannulas operate. The piston translates to a point where it may be viewed alone or adjacent to indica to ascertain proper oxygen flow to the user, by a caretaker from a distance.

## Description

### BACKGROUND OF THE INVENTION

### 1. Field of the Invention

This application claims the benefit of U.S. Provisional Application Serial Number 60/780,980, filed on March 10, 2006. The present invention relates to flow meters which provide a visual signal for gas flow through a conduit. More particularly it relates to a flow meter which provides a visual cue viewable with the human eye, as to the flow of gas through a cannula which conventionally employs very low pressure and gas volume to a patient wearing it. The device is adapted to be engaged between the nose or mouth-mounted cannula and a compressed oxygen supply delivered to the cannula through a flexible conduit. It delivers an easily read confirmation of actual continuous flow and of volume of oxygen.

### 2. Prior Art

Flow meters are frequently employed to show gas flow through a conduit from a compressed source to a destination for the gas. Generally, some movement in the meter of the meter components allows inspection of the meter to affirm gas flow during use. However, in the vast majority of such uses, the volume and pressure of the gas being transmitted from a high pressure source is sufficient to provide movement to some type of mechanically activated meter or visual cue as to the volume and pressure of the gas communicating from the high pressure source through the conduit supplying the user or using device.

Conventional flow meters for breathing apparatus typically employ a ball which translates back and forth inside an appropriate sized conduit. The moving ball is visible from a distance such as adjacent to the meter to confirm gas flow. Such meters as the *Nelson* flow meter typically employ the ball inside a tapered tube about 2 ½ inches long. Along the side of the tube are scale(s) marked in the altitude. The meter is used in combination with a valve that is situated at the base of the flow meter that allows for adjustment of the flow of oxygen.

To operate the typical flow meter, the user must hold the unit vertically and then adjust the needle valve to locate the floating ball to a stationary position next to the appropriate altitude scale. In cases of variable altitude such as in an aircraft, the flow rate is readjusted accordingly to allow for the proper flow of oxygen in relation to the amount in the ambient air. After the setting is made, the flow meter can be placed in any position; however, once placed in a non-vertical position, it does not work and does not show if the oxygen is actually flowing. Consequently, a user or a caretaker cannot easily see if there is a flow itself, and on closer inspection whether there is a flow of gas that is sufficient to provide for the health of a patient or user. It is also hard for people responsible for user health, such as a flight attendant on an airplane standing adjacent to the user, to ascertain if oxygen is actually flowing to the passenger. In such situations there is no continual visual cue to the user or caretaker to ascertain the actual flow of oxygen to the user, and on closer inspection whether there is a sufficient flow rate. Such can be extremely hazardous to the user since a very short time without oxygen can cause death or unconsciousness.

Other types of flow meters exist for gas from a pressurized supply to a user or device. Many such devices use electronic sensors or metering components to measure the amount and force of gas flow through a conduit from a higher pressure source to a lower pressure destination. However, these meters suffer the same problem as the ball type meter noted earlier in that a user connected and breathing from the high pressure source cannot continually ascertain if there actually is flow in the low presser and volume in a conduit supplying a breathing mask. A caretaker or third party consequently has the same problem ascertaining if the user is getting oxygen when needed, which can result in serious injury when the user is a hospital patient or otherwise ill individual badly in need of continuous oxygen supplementation.

As such, there is a continuing unmet need for an improved oxygen or gas flow meter which will operate in the low pressure conduit between a cannula and an oxygen source. Such a device in such operation should provide continuous real time easily-viewed feedback as to actual flow through the conduit of oxygen to the user. Such a device should be easy to read in order to ascertain there is actual flow to the patient or user from a distance by third parties or close-up by the user or patient. Still further, such a device should have sufficient visual cues or indica thereon, and sufficient travel during use, to allow a caretaker standing across the room or next to the patient's bed to easily confirm an actual flow and a sufficient and continuous oxygen flow from a supply and to the cannula worn by the patient. Further, such a device should be easy to engage in the supply conduit from the oxygen source to the cannula used to the nose or over the mouth, or be engageable to the cannula itself.

Still further, such a system should have a display and travel of a gauge that is large enough to provide the visual cues of oxygen flow continuously, no matter what direction of orientation the device occupies, be it vertical, angled, or horizontal. Only then can patients, airline passengers, and others who vitally depend on supplemental oxygen be rendered safe from unnoticed dangerous interruptions to their breathing air which can easily go unnoticed with current flow meters now being employed.

### SUMMARY OF THE INVENTION

The device and method of employing it herein disclosed and described achieves the above-mentioned goals through the provision of a pneumatic operated, biased piston, with sufficient travel in a viewable cavity, to provide a visual indicator of very low pressure and volume levels employed in the oxygen supply to a cannula or mask. In a preferred mode it is adapted for insertion in the flexible conduit supplying a face-mounted cannula, nose mounted dispenser, or face mounted oxygen mask on a patient. A substantially transparent body portion of the device, having indica located thereon, operates as a frame of reference in combination with a translating piston to provide a visual depiction of actual flow of oxygen in the system from the pressurized supply to the low pressure mask or cannula outlet.

An easily viewed and understood visual signal is provided by a unique cylindrical piston adapted for translation inside a cylindrical body portion even under extremely low pressures used in patient and similar face-mounted oxygen supply devices. The device thus operates at very low pressures and odd angles where current mechanical complicated electronic components would fail.

The body of the device employs a transparent wall surface or at least one portion of the wall surface being transparent to define a central cavity. This transparent surface area allows for viewing of the continually translating position of the piston in the central cavity, in relation to indicia which is fixed in position on the body as a frame of reference to the movement and relative position of the piston.

Translation of the piston by the oxygen supply, under the very low pressures and volume used in a conventional cannula and face mounted supplemental oxygen supply apparatus, is achieved by a novel and unique interaction of body and piston components. The piston is adapted on one side surface for engagement with a biasing means housed inside the body, such as a spring. The biasing means continually biases the piston in the direction of the higher pressure supply feeding the conduit. On the opposite side surface of the piston, in all the current preferred modes of the device, is formed a bowl-shaped or concave surface which dips below the circumferential edge of the piston toward the biased end of the piston. The concave surface forms a pocket on the intake side of the piston which serves as a means to increase the effective surface area on the side of the piston which communicates with the incoming oxygen supply provided to an intake side of the body.

An intake conduit communicates axially from the intake side of the body into the central chamber occupied by the translatable piston. The intake conduit in current preferred modes of the device, is frusto conical in that it narrows or tapers from a widest point to a narrow point. The widest diameter of the intake conduit is situated where it communicates in sealed engagement with the tube or other conduit communicating with the pressurized oxygen supply. The narrowest point of the intake conduit is positioned where it communicates with the central chamber housing the translating piston. This narrowing of the intake conduit thereby provides a lens to focus the stream of the pressurized oxygen upon a center point on the concave surface of the piston. This tapering at a constant decreasing cross section of the intake conduit also serves to increase the directed speed of the focused fluid stream of oxygen that communicates from the intake conduit onto the center of the depressed concave surface of the piston, taking advantage of the venturi effect.

By employing this unique arrangement of a continuously narrowing of the intake conduit for increased speed of the fluid stream and focusing the high speed fluid stream on the center portion which is the most recessed of the concave surface and employing the increased dimensional area provided by the concave surface, along with the capturing ability for the fluid stream force that concave surface provides, the assembled device is able to operate within the very low pressure and low volume used in a typical medical cannula. Further, the translating biased piston engaged inside the central cavity allows the device to operate at virtually any angle. This is very important since humans have a tendency to move about and move their heads to an infinite number of angles which would render ball-type flow meters inoperative.

Oxygen entering the central cavity of the body from the narrows cross section of the intake aperture first strikes the concave surface and then communicates past the side surface of the piston to a second end of the central cavity. The second end of the central cavity communicates with an exhaust conduit. The exhaust conduit has no need for narrowing.

The exterior of the body, at both the intake and exhaust ends, is adapted for frictional or other sealed engagement with the inside of the axial cavity of conventional flexible cannula or oxygen mask supply tubing. This renders the device easily engageable with the vast majority of the installed base of conventional hospital and airline cannula-type devices, which operate to supply users with a low pressure, low volume oxygen supply, with little or no modification to existing systems.

With respect to the above description, before explaining at least one preferred embodiment of the herein disclosed invention in detail, it is to be understood that the invention is not limited in its application to the details of construction and to the arrangement of the components in the following description or illustrated in the drawings. The invention herein described is e capable of other embodiments and of being practiced and carried out in various ways which will be obvious to those skilled in the art. Also, it is to be understood that the phraseology and terminology employed herein are for the purpose of description and should not be regarded as limiting.

As such, those skilled in the art will appreciate that the conception upon which this disclosure is based may readily be utilized as a basis for designing of other structures, methods and systems for carrying out the several purposes of the present disclosed device. It is important, therefore, that the claims be regarded as including such equivalent construction and methodology insofar as they do not depart from the spirit and scope of the present invention.

It is an object of this invention to provide an improved metering device which operates at any angle and provides constant visual confirmation, readable by human sight, of oxygen flow to the patient or user.

It is an object of this invention to provide such a device that will provide such a visual cue confirming oxygen flow that can be easily seen from across a room or from by a person standing adjacent to a patient in a bed.

It is a further object of this invention to provide such a device that is capable of insertion into conventional flexible tubing supplying cannulas and breathing masks and the like and that will operate at the very low pressure and flow characteristics of such a personal oxygen supplement supply.

It is yet another object of this invention to provide such an oxygen monitoring device capable of operating at very low pressures and rates of flow, that may be adjusted by assembly of engageable parts, to perform with the intended supply system.

These together with other objects and advantages which become subsequently apparent reside in the details of the construction and operation as more fully hereinafter described and claimed, reference being had to the accompanying drawings forming a part thereof, wherein like numerals refer to like parts throughout.

### BRIEF DESCRIPTION OF DRAWING FIGURES

Figure 1 depicts a perspective view of the gas flow metering device showing the body in transparent material and the internal components and arrangement.
Figure 2, is a side view through line 2-2 showing the concave surface of the piston and narrowing end of the conduit for increasing fluid speed and focusing incoming gas upon a central portion of the concave surface.
Figure 3 is a perspective view of the opposite end of the device from figure 1 and showing the piston in a translated position indicating a proper supply of oxygen to the patient.
Figure 4 depicts a mode of the device that is adapted to be assembled or disassembled as needed to allow for customization to the intended installation.

### DETAILED DESCRIPTION OF THE PREFERRED EMBODIMENTS OF THE INVENTION

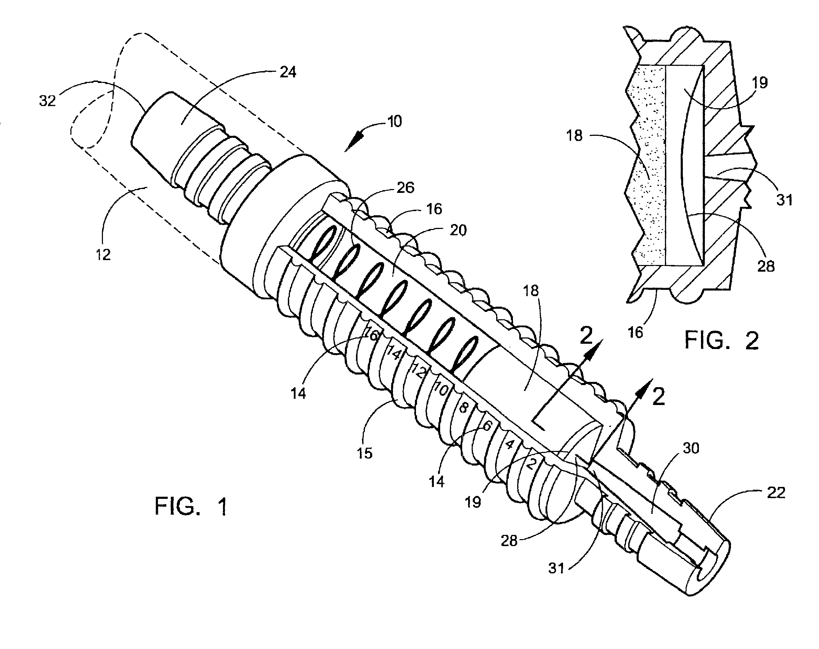

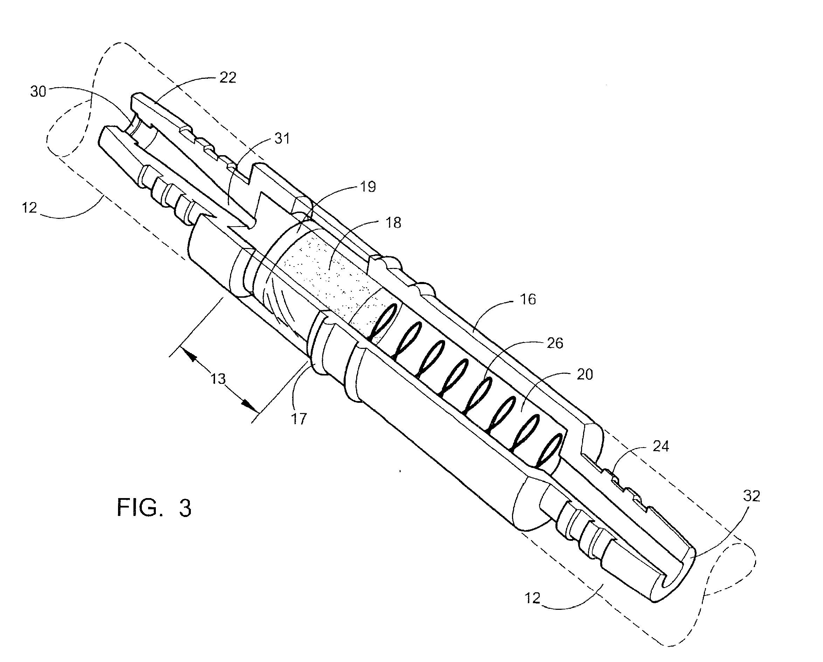

Referring now to the drawings of figures 1-4 of the disclosed device 10 which provides a pneumatic operated analog visual indicator for oxygen flow between a cannula or other face worn breathing supplementation component for a user (not shown) and a pressurized gas or oxygen supply supplying the mask, cannula, or nose dispensing component.

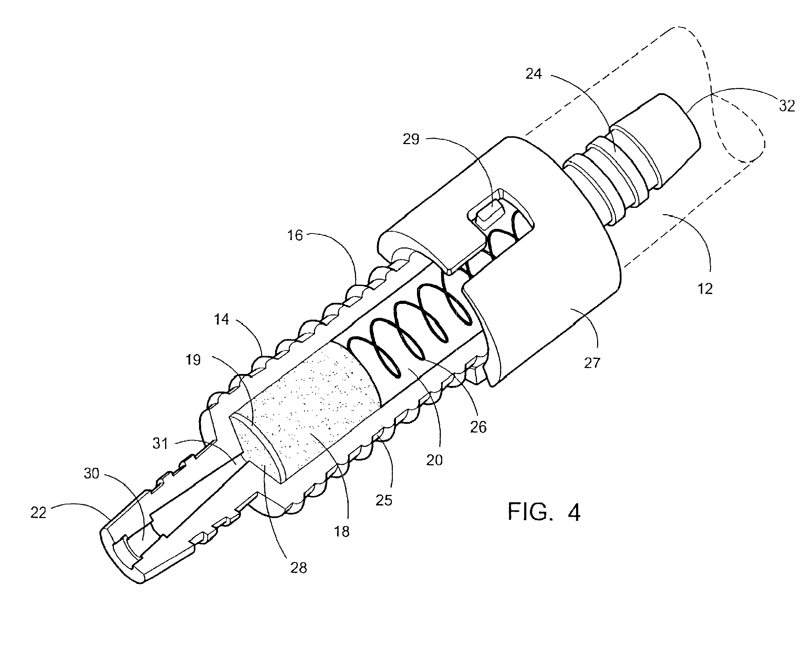

The device 10 is adapted for sealed engagement by insertion in the flexible conduit 12 supplying a face mounted cannula or oxygen mask, or nasal cannula, worn by most patients or airline passengers requiring supplemental oxygen. Indica in the form of letters or numbers 14, lines, and the like, is operatively positioned along exterior wall surface forming the substantially transparent body 16, or of the body with a transparent portion 13. The body 16 can be of unitary construction or multiple pieces as shown in figure 4. The indicia can be one or a combination of indicia indicators from a group consisting of engraved surfaces forming numbers 14 or letters, into the body 16, projections 15 on the wall of the body 16 forming letters, numbers, or ridges, and printed, silk screened, or other imprinted numerals 14 or otherwise imparted to the body 16. Those skilled in the art will realize that there are many means for imparting indica on a plastic component and all are anticipated within the scope of this invention.

The indicia in combination with a translating piston 18 and all or a portion 13 of the body 16 being transparent, provides a means to indicate the flow of oxygen or other gas through the central cavity 20 of the device 10, from an intake end 22 to an exhaust end 24, where it is communicated through flexible tubing 12 to the face mask or cannula on the patient. By colorizing the piston 18 or stripe 19 to be easily viewable in relation to the stationary indicia on the transparent body 16, its position and movement through the transparent body, relative to the indicia, is easily viewable from positions adjacent to the device, or even across a room or aisle. In a favored mode of the device 10, a portion 13 of the body 16 is transparent, in an area that the piston 18 or stripe 19 on the piston 18 occupies when there is sufficient flow incoming from the oxygen supply to translate the piston 18 thereby allowing a view of the stripe 19 or piston 18 through the transparent portion 13. In this fashion, if a user or caretaker or third party can see the stripe 19 or a colorized portion or piston 18, they will immediately know there is oxygen being supplied to the cannula attached since they can see the colorized portion of the piston 18. Conversely, if they cannot see the stripe 19 or colorized portion of the piston 18, they will immediately know that something is amiss and to check the oxygen supply. Testing has shown such viewing can be accomplished when the viewer is three feet or more away from the device 10. Thus, when oxygen is flowing through the device 10 to the cannula or mask on the user, that flow can be continuously monitored by caretakers or responsible persons from a position adjacent to the user or from a distance of three feet or more, by simply looking for the colorized piston 18 or stripe 19 being in the appropriate position.

Preferably, the piston 18 or a portion such as a stripe 19 around its exterior side surface, is colorized with a bright color such as red or florescent green or orange, or other easily viewed color, which can easily be seen through the transparent wall of the body 16. Position, and/or movement of the piston 18, translating in the central cavity 20, relative to the indica on the transparent body 16, and/or a transparent section of the body 16, can be used by the viewing person, to ascertain proper flow to the user, from a position close to the user, or from across a room. This allows medical personnel or other persons responsible for user health and safety to ascertain an oxygen supply problem before the patient or user passes out or is otherwise injured.

The piston 18 is adapted for translation inside the central cavity 20 of the body 16 to provide the above-noted visual cue of proper flow to the user when viewed in relation to the indicia as noted. The piston 18 is adapted on a first side surface closest to the exhaust 24 for engagement with a biasing means such as a spring 26. The spring 26 is calibrated to yield the proper biasing against the pressurized gas from the intake end 22 in relation to the indicia markings, to allow translation to a position where it is visible such as transparent section 13 or adjacent to indicia, either thereby showing the proper flow of gas through the central cavity 20 and onto the patient or user under the low supply pressures (15 to 22 psi) and conventional low flow rates (1 to 15 liters a minute), of cannula type devices. The supplied pressure being 15 to 22 pounds on average, is just above air pressure at sea level and hard to constantly monitor accurately with most gauges especially at odd angles the devices assume during use. The biasing spring 26 thus has sufficient bias to place the piston 18 in positions relative to the indica, to show at least a flow of gas, and preferably, at what rate even at the very low pressures and rates of flow of such cannula devices. It is most important also that the weight of the piston 18 is light enough not to move or compress the spring 26. This is because the device 10 is intended to operate at any angle during use. Currently the preferred mode of the device 10 features a piston weight of approximately .0022 pounds biased by a spring 26 with a force of .01 pounds per square inch.

Of particular importance to the function of the device 10 are the dimensional characteristics of the endwall 28 of the piston 18, opposite the spring 26 and closest to the intake 22. This is because the very law pressures of cannula supplies do not generally provide sufficient force to translate the piston 18 in an operative fashion and especially a sufficient distance of travel that can be viewed easily from a distance by their parties to affirm from a distance that oxygen is being supplied to the patient or user. Currently it was determined that approximately .25 inches is sufficient to be viewed from a distance by a caretaker or third party to ascertain flow of oxygen to the patient or user and consequently, that would be a preferred minimum distance. Of course making the central cavity longer to allow more movement can also be done but requires the device be longer also which may not be desirable in some instances.

In all preferred modes of the device 10, the endwall 28 is dimensioned to form a depression the depth of which is defined by a concave surface of the endwall 28. This is most important to the functioning of the device 10 as experimentation has found it to allow the device to operate in the low pressure, low volume environment, of a cannula oxygen supply and translate the piston 18 so it can be viewed. The concave surface of the endwall 28 is most important because it markedly increases the surface area of the endwall 28 on which the incoming stream of pressurized gas can exert force, to thereby translate the piston 18 back and forth against the resistance of the spring 26 or other biasing means, for the distance of travel of the piston where movement, or a properly stationary piston, can be easily seen from a distance or at least when standing next to the user, to confirm oxygen flow to the patient or user. Enhancing the piston 18 for easy viewing from a distance also aids in this endeavor as noted herein and is preferred in the best mode of the device 10.

Further enhancing the force generated by the incoming gas flow upon the endwall 28 and thus the translation of the piston 18 is the tapering dimensional characteristics of the intake conduit 30 which communicates axially from the intake side 22 of the body 16 into the central cavity 20 housing the translating piston 18. The intake conduit 30 in the current preferred modes of the device 10, tapers in size from a widest point, where it communicates in sealed engagement with the pressurized oxygen supply, to its narrowest point, where it communicates with the central cavity 20 at substantially the center axis of the body 16 and the cavity 20. As shown in the figures, the narrowing is achieved by a continuous reduction in size of the intake conduit 30 from its widest point to the narrowest point in a frusto conical shape. This narrowing of the intake conduit 30 thereby provides lens or means to focus the pressurized oxygen or gas stream, entering the central cavity 20 directly upon a center portion of the concave surface of the endwall 28 of the piston 18. In addition to focusing the force of the gas at the lowest point on the depressed concave endwall 28, the narrowing of the intake conduit 30 also provides a means to increase the speed of the directed pressure stream from the intake conduit 30 onto the endwall 28 provided by the depressed concave surface on the end of the piston 18. This unique combination of a narrowing of the intake conduit 30 providing a focusing and acceleration of the gas stream, upon the enlarged surface area of the endwall provided by the depression, increases the force imparted to translate the piston and thereby significantly increases the ability of the device to function in the low pressure, low volume requirements of a patient oxygen supply. This increase in directed force thereby allows sufficient movement of the piston 18 a distance along the central cavity to provide visual confirmation of flow. Translated this distance, the piston viewed through the sidewall thereby provides an easily readable signal or meter that can be viewed and confirmed from a distance by caretakers. Further, unlike conventional ball type flow meters, the device 10 using a translating piston 18 will operate at any angle the user is likely to position it.

Oxygen entering the central cavity 20 from the intake conduit 30 once focused on the depression in the endwall 28 will rebound from the endwall 28 and thereafter communicate around the endwall 28 of the piston 18 and through a gap formed between the side surface of the piston 18 and the wall of the central cavity 20. This gap is sized to allow the gas flow past the piston, while still allowing the piston 18 to operatively slide or translate in the central cavity 20. The optimum gap in the current preferred mode of the device is between .013 to .017 inches. Preferably, the diameter of the piston 18 is sized to occupy between 85-95 percent of the diameter of the central cavity with experimentation showing the current best mode of the device occupying approximately 90 percent of the cross sectional area of the central cavity 20. By allowing 10 percent of the cavity around the piston 18 for communication of gas past the piston 18 to the patient or user, sufficient area for passage is provided while concurrently the piston is sufficiently supported inside the central cavity in a manner that prevents binding during translation. The oxygen thereby progresses to a second end of the central cavity 20 closest to the exhaust end 24 of the body 16 and then communicates with an exhaust conduit 32 to the cannula or face mask.

The exterior of the body 16 at both the intake 22 and exhaust 24 ends, is adapted for frictional sealed engagement inside the axial cavity of conventional flexible tubing 12 used for cannula oxygen supply. Of course other means for sealed engagement may be employed by those skilled in the art and such are anticipated.

The device 10 employing the improved focusing of incoming gas and increased velocity of the incoming gas stream speed from the narrowing of the intake conduit, and increased endwall surface catching the focused higher speed gas supply, operates in conjunction with indicia and piston translation to provide a visual cue or confirmation continuously of oxygen flow at virtually any angle. The piston during operation will move to a static position when no breath is taken and will translate back and forth slightly when a breath is taken. The device may be formed of a unitary or fixed body 16 or of a plurality of assembleable components as in figure 4.

When provided as in figure 4, the device would feature a body 16 which is removably engageable to an endcap 27. As shown in a particularly preferred mode of the device 10 the endcap 27 is adapted for a bayonet mount using projections 29 on the outside of the body 16 which is engaged in a slot in the endcap 27. Force from the spring 26 against the inside of the endcap 27 maintains the projection 29 firmly mounted against the endcap 27. A means to vary the biasing force of the spring 26 may also be provided in this mode of the device by spacing the endwall of the endcap 27 engaging the spring 26 closer or further away from the piston 18.

Additionally, as a kit of components that may be assembled, the device 10 will allow for on-site customization for both tubing diameter for the oxygen supply pressure employed at the site. Means of adapting the device to operate with individual incoming pressure characteristics at a particular location is provided by one or a combination of changing the biasing force of the internal spring 26 by using a spring with differing force, or by changing the endcap 27 to one with an endwall further or closer to the piston 16, or by providing a body 16 with differently spaced indica 14, or by employing a piston 18 with a smaller or larger depression and thus varied area of impact for the incoming focused gas stream, or changing the size of the smallest portion of the intake conduit 30, or by changing the diameter of the piston 16 and central cavity 20 to provide more or less surface area for the depression, to adapt the device 10 to work for the purpose intended and with the gas pressure provided and the cannula or face mask to be used. In such cases the device 10 can be provided as a kit having a plurality of springs 26 with different biasing rates, a plurality of pistons with different depression depths, a plurality of endcaps 27 providing different spacing of the engagement of the spring 26 from the piston 18, or a plurality of bodies with different sized intake conduits 20. Changing any one or combination of the components will change the operational characteristics of the assembled device 10.

While all of the fundamental characteristics and features of the invention have been shown and described herein, with reference to particular embodiments thereof, a latitude of modification, various changes and substitutions are intended in the foregoing disclosure and it will be apparent that in some instance, some features of the invention may be employed without a corresponding use of other features without departing from the scope of the invention as set forth. It should also be understood that various substitutions, modifications, and variations may be made by those skilled in the art without departing from the spirit or scope of the invention. Consequently, all such modifications and variations and substitutions are included within the scope of the invention as defined by the following claims.

## Claims

1. An apparatus for metering gas flow to a facemask or cannula from a pressurized gas supply, comprising:
a body having a first end and a second end and body sidewall communicating therebetween;
a central cavity extending axially between said body sidewall and said first and second ends, said cental cavity having a diameter;
an intake conduit communicating from a first aperture, through said first end, to a second aperture communicating with said central cavity;
an exhaust conduit communicating through said second end to said central cavity;
means for sealed communication of said intake conduit with a pressurized gas supply;
said exhaust conduit adapted for sealed engagement with a cannula or face mask;
a piston having a side surface extending between a first end and an endwall, said endwall having a surface area;
said piston dimensioned for translatable engagement in said central cavity;
said endwall positioned closest to said intake conduit when in said translatable engagement;
means to impart a biasing force to said piston to translate said piston toward said intake conduit to a biased position;
said intake conduit having a narrowing diameter extending from a widest point at said first aperture, to a narrowest point at said second aperture;
said narrowing diameter providing a means to focus said pressurized gas supply communicated from said second aperture, in a gas stream communicating substantially with a central portion of said surface area of said endwall;
said gas stream communicating with said central portion thereby providing a translating force sufficient to overcome said biasing force and translate said piston to an operating position a distance toward said exhaust conduit from said biased position;
said piston viewable through a substantially transparent section of said sidewall of said body, when translated to said operating position; and
said piston, when viewable, providing means to visually confirm communication of said pressurized gas supply from said intake conduit, to said exhaust conduit.

2. The apparatus for metering gas flow of claim 1 additionally comprising:
said surface area of said endwall defining an endwall having a bowl shaped depression;
said depression providing means to increase said surface area of said endwall thereby providing means to increase said translating force; and
said means to increase said translating force providing means for operation of said apparatus at low pressure levels of said gas supply.

3. The apparatus for metering gas flow of claim 1 additionally comprising:
said piston having a weight; and
said means to impart a biasing force being a spring with said biasing force exceeding said weight; and
said apparatus operable at any angle to accurately provide said means to visually confirm communication of said pressurized gas supply from said intake conduit, to said exhaust conduit.

4. The apparatus for metering gas flow of claim 2 additionally comprising:
said piston having a weight; and
said means to impart a biasing force being a spring with said biasing force exceeding said weight; and
said apparatus operable at any angle to accurately provide said means to visually confirm communication of said pressurized gas supply from said intake conduit, to said exhaust conduit.

5. The apparatus for metering gas flow of claim 1 additionally comprising:
at least a portion of said side surface of said piston being colorized providing means to view said piston in said operating position, from a viewing position at least 3 feet away from said body.

6. The apparatus for metering gas flow of claim 2 additionally comprising:
at least a portion of said side surface of said piston being colorized providing means to view said piston in said operating position, from a viewing position at least 3 feet away from said body.

7. The apparatus for metering gas flow of claim 4 additionally comprising:
at least a portion of said side surface of said piston being colorized providing means to view said piston in said operating position, from a viewing position at least 3 feet away from said body.

8. The apparatus for metering gas flow of claim 2 additionally comprising:
said narrowing diameter of said intake conduit being a constant reduction in diameter from said widest point to said narrowest point thereby forming said intake conduit in a frusto conical shape.

9. An apparatus for metering gas flow to a facemask or cannula from a pressurized gas supply, comprising:
a body having a first end and a second end and body sidewall communicating therebetween;
a central cavity extending axially between said body sidewall and said first and second ends, said cental cavity having a diameter;
an intake conduit communicating from a first aperture, through said first end, to a second aperture communicating with said central cavity;
an exhaust conduit communicating through said second end to said central cavity;
means for sealed communication of said intake conduit with a pressurized gas supply;
said exhaust conduit adapted for sealed engagement with a cannula or face mask;
a piston having a side surface extending between a first end and an endwall, said endwall having a surface area;
said piston dimensioned for translatable engagement in said central cavity;
said endwall positioned closest to said intake conduit when in said translatable engagement;
means to impart a biasing force to said piston to translate said piston toward said intake conduit to a biased position;
said surface area of said endwall defining an endwall formed as a bowl shaped depression;
said pressurized gas supply when communicated through said intake conduit providing a gas stream which when communicating with said endwall imparts a translating force to said piston, said translating force being in the opposite direction of said biasing force;
said depression providing means to increase said surface area of said endwall thereby providing means to increase said translating force to a level sufficient to overcome said biasing force and translate said piston to an operating position a distance toward said exhaust conduit from said biased position;
said piston viewable through a substantially transparent section of said sidewall of said body, when translated to said operating position; and
said piston, when viewable, providing means to visually confirm communication of said pressurized gas supply from said intake conduit, to said exhaust conduit.

10. The apparatus for metering gas flow of claim 9 additionally comprising:
said intake conduit having a narrowing diameter extending from a widest point at said first aperture, to a narrowest point at said second aperture;
said narrowing diameter providing a means to focus said gas stream to communicate substantially with a central portion of said surface area of said endwall;
said narrowing diameter providing means to increase a velocity of said gas stream; and
said focus of said gas stream on said central portion and said increase of said velocity providing means to increase said translating force to overcome said biasing force, whereby said apparatus for metering gas flow will operate when said pressurized gas supply is communicated at pressures between 15 and 22 psi.

11. The apparatus for metering gas flow of claim 10 wherein said intake conduit narrows in said diameter at a constant rate along a distance from said first aperture to said second aperture; and
said intake conduit is frusto conical in shape thereby providing enhanced focusing of said gas stream and enhanced increase in said velocity of said gas stream.

12. The apparatus for metering gas flow of claim 2 additionally comprising:
said means for sealed communication of said intake conduit with a pressurized gas supply said first end of said body dimensioned to sealably engage with a flexible tube;
said exhaust conduit is adapted to engage with said cannula through dimensioning said second end of said body to sealably engage with a flexible tube communicating with said cannula.

13. The apparatus for metering gas flow of claim 3 additionally comprising:
indicia on said sidewall viewable in combination with said piston to ascertain a flow rate of said pressurized gas, through said apparatus.

14. The apparatus for metering gas flow of claim 10 additionally comprising:
indicia on said sidewall viewable in combination with said piston to ascertain a flow rate of said pressurized gas, through said apparatus.

15. The apparatus for metering gas flow of claim 3 formed of assembleable components comprising:
said body formed of an endcap and a body portion having a first side and said second end and having said central cavity formed therein;
said endcap removably engageable to said first end of said body portion, to an engaged position;
said endcap forming said first end of said body when in said engaged position;
said spring engageable in said central cavity when said endcap is removed; and
said piston engageable in said central cavity when said endcap is removed.

16. The apparatus for metering gas flow of claim 15 additionally comprising means of adapting said apparatus to operate different pressure levels of said pressurized gas supply and properly translate said piston, comprising one or a combination of:
Changing the biasing force of the said spring by using a spring with differing force, changing the endcap to one with an endwall further or closer to the piston, employing a body portion with differently spaced indica, employing a piston with a smaller or larger depression and thus varied area of impact for said gas stream, changing the size of the narrowest point of the intake conduit, or by changing the diameter of the piston and central cavity in which it engages to provide more or less surface area for the depression, whereby said apparatus may be adapted to translate said piston said distance with the anticipated said gas supply pressure.

17. The apparatus for metering gas flow of claim 3 engaged directly to a cannula.
